# EUROPEAN PATENT SPECIFICATION

(11) **EP 3 047 853 B1**
(45) Date of publication and mention of the grant of the patent: **15.05.2019**
(21) Application number: 16152839.3
(22) Date of filing: 26.01.2016
(51) Int. Cl.: A61K 35/57, A23L 33/18, A23L 13/50, A61K 35/744

(54) **BROTH COMPOSITIONS AND THEIR USE AS PREBIOTICS**
BRÜHENZUSAMMENSETZUNGEN UND DEREN VERWENDUNG ALS PRÄBIOTIKA
COMPOSITIONS DE BOUILLON ET LEUR UTILISATION EN TANT QUE PRÉBIOTIQUES

(30) Priority: 26.01.2015 US 201562108008 P
(43) Date of publication of application: 27.07.2016
(60) Divisional application: 19160359.6
(73) Proprietor: Lynch, Stephanie, Springfield, MO 65804 (US); Dake, Roger L., Springfield, MO 65810 (US); Durham, Paul L., Nixa, MO 65714 (US); Norton, Rhy, Ozark, MO 65721 (US); Hawkins, Jordan L., Springfield, MO 65802 (US)
(72) Inventor: Lynch, Stephanie, Springfield, MO 65804 (US); Dake, Roger L., Springfield, MO 65810 (US); Durham, Paul L., Nixa, MO 65714 (US); Norton, Rhy, Ozark, MO 65721 (US); Hawkins, Jordan L., Springfield, MO 65802 (US)
(74) Representative: m patent group

(56) References cited:
- WO-A1-2013/080911
- CN-A- 103 564 516
- US-A1- 2004 005 382
- US-A1- 2013 345 139
- US-A1- 2015 011 500
- FEDERICA MELI ET AL: "Effect of Protein Hydrolysates on Growth Kinetics and Aminopeptidase Activities of Lactobacillus", CURRENT MICROBIOLOGY, vol. 68, no. 1, 29 August 2013 (2013-08-29), pages 82-87, XP055278156, Boston ISSN: 0343-8651, DOI: 10.1007/s00284-013-0445-z

## Description

### BACKGROUND

This disclosure relates to the field of nutritional and dietary supplement as well as the general field of microbiology.

Microorganisms are known to colonize the gastrointestinal tract of mammals. Some microorganisms have certain health benefits for the host. These beneficial microorganisms are also known as "probiotic microorganisms" or "probiotics." Beneficial bacteria (or "good" bacteria) are also referred to as "probiotic bacteria." Some probiotic microorganisms may exist naturally in the gut of the host. Others may be introduced into the host by dietary supplement.

The term "prebiotic" generally refers to substances that induce the growth (and hence the live count or CFU) or activity of probiotic microorganisms (*e.g.,* bacteria or fungi). However, most, if not all prebiotics are carbohydrate based and may contain glutenfrom various plant sources.

US 2013/345139 A1 relates to a composition for nutritional purposes with reportedly has an improved efficacy in relation to the build-up of the bone matrix, comprising about 1 to about 99% by weight of collagen hydrolysate and about 1 to about 99% by weight of one or more prebiotics, in each case based on the dry mass of the composition.

US 2015/011500 A1 discloses broth compositions prepared from poultry. Selected poultry raw materials are processed to obtain a broth having high protein content. Certain specific amino acids are reportedly present in relatively higher concentration as compared to home-made broth and other commercial products. The disclosed broth compositions are reported to be effective in preventing and/or treating joint diseases and may also provide other nutritional and health benefits.

Federica Meli et al., Curr. Microbiol. 68, 2013, 82-87, report on the effect of protein hydrolysates on growth kinetics and aminopeptidase activities of *Lactobacillus*.

### SUMMARY

The present disclosure advances the art by providing a composition for use in a method of providing health benefits to an individual by enhancing the microbiome of said individual by enhancing one or more probiotic bacteria in said individual according to claim 1. The method comprises administering to said individual a composition in an amount effective in enhancing said one or more probiotic bacteria. According to the present invention, said composition comprises soluble collagen fiber prepared from poultry, and said composition is obtainable by a method comprising the steps of mechanically separating and comminuting poultry parts with bones and cartilage to fine pieces of less than 5 mm, and cooking the pieces at a temperature from 70 to 150°C for a period from 8 minutes to 6 hours or longer. More particularly, in one embodiment, the disclosed prebiotic composition is gluten-free. Preferred embodiments are the subject of the dependent claims and the description that follows.

According to the present invention, the composition is be prepared from poultry, such as chicken, turkey, or other birds.
In another embodiment, the disclosed composition may be in the form of broth, stock, extract, or powder.

As mentioned, the composition according to the present invention is provided for use in a method for enhancing the microbiome of an individual by enhancing one or more probiotic bacteria in said individual, the method comprising administering to the individual an effective amount of a composition prepared from poultry parts. In one aspect, the effective amount is an amount effective in enhancing at least one probiotic bacterium of the individual. In another aspect, enhancing a probiotic bacterium means improving the well-being or increasing the live count (i.e., colony-forming unit or CFU) of the probiotic bacterium in the gastrointestinal tract (or gut) of the individual. The CFU of a bacterium in the individual may be measured directly or indirectly. Indirect measurements may include but are not limited to measuring the CFU of the bacterium in fecal samples from the individual. In one aspect, the method of metagenomic sequence counts may be used to measure the CFU of a bacterium in a sample.

In another embodiment, the effective amount to be administered may be an amount of the broth composition that increases the live count of at least one probiotic bacterium in the gut of the individual by 2-200 folds, by 5-200 folds, by 10-200 folds, by 20-200 folds, or by about 200-folds, after the composition is administered to the individual. In another aspect, the effective amount may be an amount of the broth composition that increases the proportion (or percentage) of at least one probiotic bacterium (i.e., selectively increases the count of at least one beneficial probiotic bacterium) in the gut microbiome of the individual. In another aspect, the effective amount may be an amount of the broth composition that increases the diversity of the gut microbiome in the individual.

In one embodiment, the disclosed broth composition for the use according to the present invention may be administered to the individual consecutively at the effective amount every day for 1 day, 2, 3, 4, 5, 6, 7 days, or 14 or more days. In another embodiment, the composition may be administered to the individual non-consecutively, for example, every other day over a period of 4, 6, 8, 10, 12, 14 or more days.

The composition for the use according to the present invention has a higher concentration of certain beneficial components. The composition for the use according to the present invention contains soluble collagen fibers. For purpose of this disclosure, the term "soluble collagen fiber" is used to refer to fragments of collagen protein that are soluble in a broth after being subject to the broth-making process. In one aspect, the fragments may represent full-length or partial-length of the collagen protein. In another aspect, the fragments may contain small peptides or single amino acids derived from the collagen. In another aspect, at least 20%, 30%, 40%, or at least 50% of the soluble collagen fibers contains fragments or peptides of 20 or more amino acids long. In another aspect, the soluble collagen fibers may act as prebiotics in enhancing or promoting the probiotic bacteria in the individual. In another aspect, the soluble collagen fibers are primarily derived from the cartilage of poultry. In another aspect, the soluble collagen fibers constitute at least 20% (w/w on solid basis) of the final broth composition. In another aspect, the soluble collagen fibers may constitute at least 30%, 40%, 50%, 60%, 70%, 80%, or at least 90% (w/w on solid basis), or as high as 100% of the final broth composition. In another aspect, the soluble collagen fibers may be derived from Type I, II or III collagen.

In another embodiment, certain beneficial compounds may be enriched in the disclosed compositions to a greater extent as compared to other broth products currently available on the market.

In another embodiment, one or more ingredients may be supplemented in the broth to achieve certain health benefits that are not generally associated with chicken broth. Examples of such supplements may include but are not limited to ginger, coffee extract, ginseng, green tea, other botanicals such as willow bark or boswellia, curcumin/turmeric, omega-3 fatty acids, fish oil, krill oil, algal oil, Pycnogenol French maritime pine bark extract, grape seed extract, flax seed extract, ribonuclease, angiogenin, lactoferrin, ribonuclease-enriched lactoferrin, S-adenosyl methionine, collagen, collagen proteins (e.g., Type I, II or III collagens) or collagen peptides, gelatin, avocado/soybean unsaponifiables (ASU), extract of hops cones, egg shell membrane, polypeptides derived from milk, such as casein or whey, MSM (Methylsulfonylmethane), Yucca, Devils claw, Bromelain, glutamic acid, cocoa, stinging nettle, Vitamin E, Vitamin D3, walnut extract, etc. In one aspect, the disclosed broth may contain significant amount of collagen, collagen proteins and/or collagen peptides. In another aspect, significant amount of the collagen, collagen proteins and/or collagen peptides may be present naturally in the disclosed broth. In another aspect, significant amount of the collagen, collagen proteins and/or collagen peptides may be added into the disclosed broth as a supplement.

In another embodiment, according to the methods in which the composition is to be used according to the present invention, an individual who is in need of increased count of a probiotic bacterium is first identified before the broth composition is administered to the individual. Such individuals who are in need of the disclosed broth composition may include but are not limited to those who have unbalanced microbiome in their GI system, and those who need higher number (CFU) of certain desirable probiotic bacteria, such as lactic acid producing bacteria. Examples of such desirable bacteria may include but are not limited to *Lactobacillus, Parasutterella, Morganella, Oscillospira, Coprococcus, Oscillibacter, Odoribacter, Gastranaerophilales, Allabaculum,* or *Faecalibacterium.* In another embodiment, one example of desirable probiotic bacteria may be *Lactobacillus rhamnosus.* In another embodiment, the broth compositions provide not only proteins (and amino acids) as nutrients, but also proteins (and amino acids) in the form of collagen fibers which help increase the number and percentage of probiotic bacteria in the gut microbiome of the individual. Thus, in one aspect of the present disclosure, administration of the disclosed broth compositions may eliminate the need for continuous supplementation of probiotics to an individual who would otherwise have to take probiotic supplement regularly. The term "regularly" means at least once a month, once a week or at least once a day.

For purpose of this disclosure, the term "enhance" means increase the growth (and hence the live count or CFU) or activity of a microorganism. In one embodiment, the disclosed composition may enhance one or more probiotic bacteria that are native in an individual. The term "native" means the microorganism naturally exist in the gut of the individual. In another embodiment, the disclosed composition may enhance one or more probiotic bacteria that are not native in an individual. Both native or non-native probiotic bacteria may be supplemented to an individual.

In one aspect, the disclosed composition may be administered to the individual by oral feeding. In another aspect, the disclosed composition may be administered in conjunction with one or more probiotic bacteria. In another aspect, the disclosed composition may be mixed with one or more probiotic bacteria and administered to an individual as a mixture.

In another aspect, the prebiotic composition is prepared according to the process specified and then be prepared into a liquid or solid (e.g., powder) form to be taken as a dietary supplement.

In one embodiment, the one or more probiotic bacteria that are enhanced by the disclosed composition may include at least a lactic acid producing bacterium. In another embodiment, the lactic acid producing bacteria may include, by way of example, a *Lactobacillus* bacterium. In another embodiment, the lactic acid producing bacteria may include *Lactobacillus rhamnosus.*

In home-style cooking, chicken or turkey broths may be made by cooking for extended period of time in an open vessel or under high temperature in a pressurized vessel. By contrast, this disclosure provides broth products having unique composition and methods of preparing the same. Also disclosed here are methods of selecting and preparing raw materials, cooking raw materials, and separating and purifying the resultant broths.

According to an embodiment not forming part of the present invention, raw materials from poultry are comminuted to a great extent to maximize extraction of various beneficial compounds. In one aspect not forming part of the invention, the raw materials may be reduced to a size of less than about 2cm, 1 cm, 5mm, 4mm, 3mm, 2mm, or less than 2mm. Mechanical processing of the poultry parts to small sized particles prior to cooking may help maximizing extraction of certain compounds. In another embodiment not forming part of the invention, because the poultry parts have been mechanically processed to very small-sized particles prior to cooking, more gentle cooking condition (e.g., at a temperature of 60-100°C) and shorter cooking time (from about 8 minutes to 300 minutes) may be used to obtain the broth from the processed poultry parts. Such gentler and shorter cooking may help prevent inactivation of certain compounds that would be otherwise inactivated if conventional processing and cooking methods are used. Preservation of such compounds may explain the superior health benefits observed when broths not forming part of the invention are tested against other broth products that are not prepared according to the instantly disclosed methods.

According to the present invention, poultry parts with bones and cartilage are mechanically separated and comminuted to fine pieces of less than 5mm (millimeter), 4mm, 3mm, 2mm, or 1 mm in size. In one aspect, no steps are taken to remove the residual meat from the bones. These small pieces are cooked at about 70°C, 100°C, 110°C, 120°C, or as high as 150°C, for at least 8 minutes, 15 minutes, 30 minutes, 1 hour, 2 hours, 3 hours, or 6 hours or longer to maximize the extraction of certain broth fraction and/or compounds.

In another embodiment, the broth prepared according to the disclosed methods show different protein profiles from those obtained from other commercial broth products when analyzed by SDS-PAGE. In one aspect, at least 10%, 20%, 30%, 40%, 50%, 70%, or 90% of the total proteins in the disclosed compositions have a molecular weight of between 10 kD (kilo-Dalton) and 70 kD. In another aspect, at least 95% of the proteins in the disclosed compositions have a molecular weight of less than 100 kD. In another aspect, the methods may be used to further reduce molecular weight of the proteins in the broth to less than 10 kD, 5 kD, or even lower than 3 KD to enhance assimilation by a subject. By way of example, one or more enzymes that digest proteins may be used to reduce the molecular weight of the proteins in the broth.

In another embodiment, the disclosed composition may contain at least 40% protein by weight of total solids. By way of example, the disclosed composition may contain 50%, 60%, 70%, or 80% or more protein by weight of total solids in the composition. In another embodiment, the disclosed composition may contain less than 10% carbohydrate by weight of total solids. By way of example, the disclosed composition may contain 8%, 5%, 2%, or 1% or less carbohydrate by weight of total solids in the composition. In another embodiment, the disclosed composition is prepared from an animal source and does not contain any proteins from a plant source. In another embodiment, the disclosed prebiotic composition does not contain detectable amount of gluten. In another embodiment, the disclosed prebiotic composition does not contain significant amount of gluten that would cause adverse reaction in an individual sensitive or allergic to gluten.

In another embodiment, the disclosed composition may contain the amino acids proline and histidine, wherein the ratio between proline and histidine is at least 4:1 by weight. In one aspect, the proline is present in the composition by at least 8% (w/w), 10% or greater on solid basis. In another aspect, the ratio between the proline and the histidine is at least 6:1 by weight.

In another embodiment, the disclosed composition may contain the amino acids glycine and histidine, wherein the ratio between the glycine and histidine is at least 6:1 by weight. In one aspect, the glycine is present in the total amino acids of the disclosed compositions by at least 12%, 14% or greater (w/w) on solid basis. In another aspect, the ratio between the glycine and the histidine is at least 10:1 by weight.

In another embodiment, the disclosed composition may contain the amino acids hydroxyproline and histidine, wherein the ratio between the hydroxyproline and histidine is at least 4:1 by weight. In one aspect, the hydroxyproline is present in the total amino acids of the compositions by at least 7%, 8% or greater (w/w) on solid basis. In another aspect, the ratio between the hydroxyproline and the histidine is at least 6:1 by weight.

In another embodiment, the disclosed composition may contain proline, glycine, hydroxyproline and histidine. In one aspect, the ratio between the glycine and the histidine is at least 6:1 by weight. In another aspect, the glycine is present in the composition by at least 12% (w/w) on solid basis. In another aspect, the ratio between the proline and the histidine is at least 4:1 by weight. In another aspect, the ratio between the hydroxyproline and the histidine is at least 6:1 by weight.

In another embodiment, the disclosed composition may contain proline and other amino acids, and the amount of the proline is at least 8%, 10% or greater by weight of the total amino acids in the composition. In another embodiment, the disclosed composition may contain glycine and other amino acids, and the amount of the glycine is at least 20% by weight of the total amino acids in the composition. In another embodiment, the disclosed composition may contain hydroxyproline and other amino acids, and the amount of the hydroxyproline is at least 8% by weight of the total amino acids in the composition.

In another embodiment, the disclosed composition may contain one or more branched chain amino acids (BCAA) (e.g., valine, leucine and isoleucine), and the BCAAs in the composition are present at a higher level than the level of BCAAs in other broth products. In one aspect, the amount of total BCAAs, including valine, leucine and isoleucine, is at least 5%, 6%, or 7% by weight of total amino acids in the composition.

In another embodiment, the broth products prepared according to this disclosure may have a moisture protein ratio (MPR) of between 999:1 and 1:999, or between 200:1 and 10:1. By way of example, a MPR of 200:1 means the product contains 200 parts water and 1 part meat (protein). In another embodiment, the broth products prepared according to this disclosure may have a moisture protein ratio (MPR) of between 150:1 and 40:1, or between 135:1 and 67:1.

Chondroitin sulfate (CS) is rich in cartilage and has been reported to be beneficial for joint health. The selection of raw materials and the process by which the broth is prepared may contribute to the relatively higher levels of chondroitin sulfate in the disclosed composition. In one embodiment of the present disclosure, the broth composition may contain at least 6%, 7%, 8%, or 10% of chondroitin sulfate by weight of total dry solids in the composition, as measured by using enzymatic digestion and LC-UV detection assay. *See e.g.,* Ji et al., Journal of AOAC International, Vol. 90, No. 3, 659-69 (2007), which is hereby incorporated by reference into this disclosure.

In one aspect, the composition may be in a liquid form ready to be consumed or it may be in a concentrated liquid form such as a stock. In another aspect, the composition may be in a solid form, such as a powder or a paste.

In one embodiment, the composition may contain one or more polyphenols, wherein the concentration of the polyphenols is at least 4,000 µg/ml GAE (Gallic Acid Equivalent) based on Folin-Ciocalteu assay.

In another embodiment, the composition may comprise amino acids glycine, proline and hydroxyproline, and wherein glycine constitutes at least 12% (w/w), proline constitutes at least 8% (w/w), and hydroxyproline constitutes at least 7% (w/w) of total amino acids of the composition. In another embodiment, the disclosed prebiotic composition does not contain detectable amount of gluten. In another embodiment, the disclosed prebiotic composition does not contain significant amount of gluten that would cause adverse reaction in an individual sensitive or allergic to gluten.

Probiotic bacteria are typically grown on culture media before they are harvested and used as dietary supplement. In one embodiment, the disclosed prebiotic composition may be used for culturing a probiotic bacterium. Probiotic bacteria may be grown on a medium containing, among others, the disclosed prebiotic composition prepared from poultry. In one aspect, the disclosed prebiotic composition contains soluble collagen fiber prepared from poultry.

The broth compositions disclosed herein may be characterized by the unique composition as described herein. The broth compositions may also be characterized by the methods by which they are prepared. Moreover, the disclosed broths are also unique in the health benefits they may provide to a subject. Subjects may be divided into two groups, one group ingests (or consumes) an effective amount of the disclosed broth product each day, while the other group ingests water, or other broth products. The various indicators may then be measured and compared between the two groups.

### BRIEF DESCRIPTION OF THE DRAWINGS

Figure 1 shows the UPGMA (Unweighted Pair Group Method with Arithmetic Mean) dendrogram from fecal samples.
Figure 2 shows the UPGMA dendrogram from caecal samples.
Figure 3 shows DGGE (Denaturing Gradient Gel Electrophoresis) profile generated for the selection of DNA bands representative of species influenced by the addition of AAC1 into the diet. Numbered bands indicate potential regions of interest. Lanes are as follows: (1) Caecal - Water; (2) Caecal - AAC1; (3) Caecal - Commercial product; (4) Caecal - Homemade; (5) Fecal - Water; (6) Fecal - AAC1; (7) Fecal - Commercial product.
Figure 4 shows that the different chicken broth products do not significantly alter the Bacteroidetes : Firmicutes ratio.
Figure 5 shows that AAC1 selects for *Proteobacteria* in the lower GI tract.
Figure 6 shows that AAC1 selects for or maintains *Lactobacillus* in the lower GI tract.
Figure 7 shows that AAC1 selects for or maintains *Lactobacillus* in the cecum.
Figure 8 shows that AAC1 increases the diversity of microbiota in the gut.

### DETAILED DESCRIPTION

Chicken soup and chicken broth have been available for human consumption for centuries. Studies have shown various health benefits of chicken soup or broth. According to conventional home-style method, chicken soup or broth is prepared by boiling whole chicken or large chicken parts in a pot of water for an extended period of time. The soup or broth prepared according to this method may not have maximized the health promoting effects of the soup or broth because some of the compounds may be lost during cooking or during processing, while other compounds may not have been extracted from the chicken parts.

In one embodiment, this disclosure provides methods of using chicken broth as a prebiotic. Prebiotics differ from probiotics (i.e., so called "good bacteria") in that prebiotics are not live organisms but are ingredients that can support or enhance probiotics. Prebiotics typically bypass the upper digestive system and reach the intestine intact after ingestion. It is also possible that prebiotics may influence bacteria in the upper GI section such that these bacteria in the upper GI section begin to ferment nutrients, which, in turn, provide metabolites that selectively support the "good bacteria" in the lower section of the gut. Once they reach the intestine, prebiotics may play an important role in fermentation by the normal gut microbiota. The fermentation process may stimulate and promote the proliferation of the "good bacteria," such as *Lactobacillus.*

As used herein, the term "probiotic" may refer to a number of microorganisms which can be taken by an individual as a supplement to benefit the individual. The term "probiotic" may also refer to a number of different microorganisms that naturally live in the body of a host and provide health benefit to the host. Examples of such desirable bacteria may include but are not limited to *Lactobacillus, Parasutterella, Morganella, Oscillospira, Coprococcus, Oscillibacter, Odoribacter, Gastranaerophilales, Allabaculum,* or *Faecalibacterium.* For instance, many *Lactobacillus* bacteria have been shown to be beneficial to human. *Faecalibacterium* bacteria have also been shown to be anti-inflammatory. See "Faecalibacterium prausnitzii is an anti-inflammatory commensal bacterium identified by gut microbiota analysis of Crohn disease patients." Proceedings of the National Academy of Sciences of the United States of America. 105(43): 16731-6 (2008).

The disclosed methods may help preserve certain components by processing chicken parts prior to cooking and by controlling the processing temperature and cooking time to maximize the extraction of beneficial compounds while, concomitantly, minimizing loss of activity due to harsh processing conditions.

Specially selected raw materials from poultry may be processed according to the disclosed methods to obtain a broth having high protein content. Certain amino acids are present in relatively higher concentration as compared to a home-made broth or other commercial products. In one aspect, as shown in various animal studies described herein, the disclosed broth compositions may prove effective in enhancing the well-being of probiotics of the host. In another aspect, the disclosed broth compositions may increase the live count of probiotics in the host. In another aspect, the disclosed broth compositions may selectively enhance certain microorganisms, but not others. In another aspect, the disclosed broth compositions may increase the diversity of microorganisms in the gut of the host.

The terms "broth" and "soup" refer to a liquid composition containing at least one solute and may also be used to refer to a ready to serve form, a concentrate, a stock in either liquid or solid form.

The poultry broth of the disclosure may contain a significant amount of different amino acids. Such amino acids may be present in the form of a protein or as free amino acids in the broth. Total amino acids include both amino acids present in the form of proteins and those present as free amino acids. For purpose of this disclosure, the ratio of different amino acids in a broth composition refers to the ratio between total amino acids.

The term "dry solid" or "solid" as used herein refers to the components of a liquid composition that remain after all free liquid is removed from the liquid composition. In the case of an aqueous broth, the free liquid is water.

The term "administer" means delivering of a material to an individual, such as by oral ingestion.

The term "not detectable" means a chemical or ingredient that may be present in such a small amount that it cannot be measured by currently available analytic instruments.

The term "subject" or "individual" is used to refer to a mammal, including a human being.

The term "substantially" means by at least 10-20%.

### Examples

The following examples are provided for purposes of illustration of the embodiments only and are not intended to be limiting. The scope of the present invention is limited by the claims. The raw materials, reagents, chemicals, and other materials are presented as exemplary components or reagents, and various modifications may be made in view of the foregoing discussion within the scope of this disclosure. Unless otherwise specified in this disclosure, components, reagents, protocol, and other methods used in the system and the assays, as described in the Examples, are for the purpose of illustration only.

### Example 1 Preparation of broth from turkey parts

In this example, turkey parts were used to prepare a broth and the overall quality and potential health benefits of the broth were determined. Briefly, raw turkey was mechanically separated and the parts were finely comminuted to less than 2mm in size to maximize extraction. The small-sized parts from the turkey were gently cooked to (91°C (195°F) in steam for about 15 minutes or less. The broth was then separated from the insoluble fraction by decanting. Freed fat in the broth was also removed from the broth by a centrifugal separator. The broth was concentrated in a commercial evaporator, chilled, and packaged for sale.

### Example 2 Preparation of broth from chicken parts

In this study, chopped chicken parts containing chicken bones and cartilage were cooked in water at a temperature greater than 121 °C (250°F) for more than 6 hours to maximize the extraction of certain broth fraction and/or compounds. The broth obtained from this process was designated "AAC1" for internal reference. More particularly, USDA inspected chopped raw chicken bones remaining after major muscles were removed were cooked in water in a large commercial stainless steel cooking tank. After cooking, the liquid broth portion was separated from the chicken solids by decanting. The broth was concentrated in a commercial evaporator then spray dried and packed in labeled containers.

### Example 3 Comparing the protein and amino acid profiles of different broths

Proteins are large molecules composed of one or more chains of amino acids that perform a wide array of functions in biological systems including, for example, functioning as enzymes, facilitating cell communication, and providing structural support to cells. Humans, as well as other animals, obtain essential amino acids from protein consumed as part of their diet since they lack enzymes needed to synthesize them. The objective of this study was to determine the concentration and size range of proteins in chicken broth AAC1 as compared to a home-made product.

One sample prepared according to this disclosure (AAC1) and another sample prepared according to home-style cooking methods (Homemade) were compared. The percent solid (w/v) for AAC1 was 8% solid, while the percentage (w/v) for Homemade was 1.7% solid.

Prior to determining the amount of protein by the Bradford method, each sample was diluted in distilled water to a final 1% w/v solution. A standard curve was prepared using bovine serum albumin (0-3.5µg/µL). All samples were analyzed in triplicate. The amount of total protein was determined using a plate reader at a wavelength of 595 nm. Results are shown in Table 1.

**Table 1 Amount of total protein in broth samples**

| **Sample** | **Values** | **Results** | **Mean Results** | **Concentration** | **SD** | **CV** |
|---|---|---|---|---|---|---|
| **AAC1** | 0.446 | 1.558 | 1.691 | 1.691 | 0.132 | 7.8 |
| | 0.461 | 1.693 | | | | |
| | 0.474 | 1.821 | | | | |
| **Homemade** | 0.544 | 2.496 | 2.52 | 2.52 | 0.034 | 1.3 |
| | 0.549 | 2.544 | | | | |

To correct for differences in the percent solids in AAC1 (8%) and Homemade (1.7%) samples, the protein values based on a 1% solution for AAC1 and Homemade were multiplied by their respective starting % solids. The final adjusted amount of total protein for AAC1 and Homemade are shown in Table 2.

**Table 2 Adjusted total protein concentration in AAC1 and homemade broth**

| **Sample** | **Adjusted Final Concentration** | **% of AAC1** |
|---|---|---|
| **AAC1** | 13.6 µg/ul | - |
| **Homemade** | 4.3 µg/ul | 31.6% |

To determine the protein profile of each sample, equal volumes of the AAC1 and Homemade samples (15.6µl) were each mixed with Laemmli's sample buffer and a reducing agent, heated at 95°C for 5 minutes, and separated on a 4-12% Bis Tris gel. The relative size range of the proteins was determined by comparison to a commercially available protein standard (ranging from 4.5 kDa to 300 kDa). A constant voltage of 150V was applied to the gel for 30 minutes, allowing for separation of proteins in each sample. The proteins were visualized in the gel using SimplyBlue™ SafeStain.

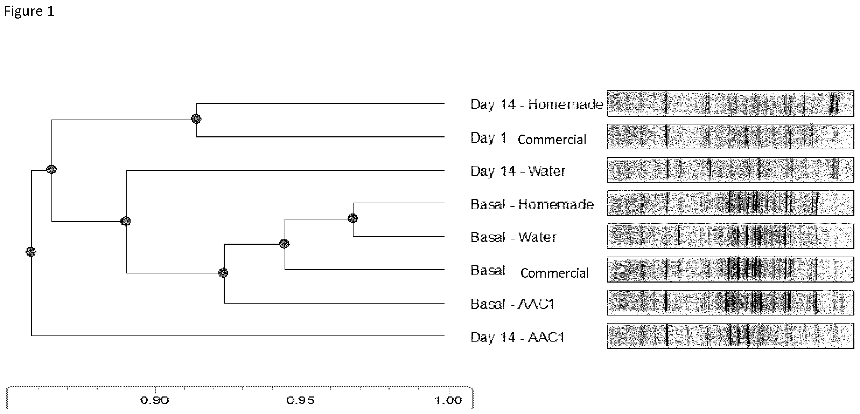

The results are shown in Fig. 1. Lane 1 shows the protein profile for homemade broth, lane 2 for the AAC1 broth, and lane 3 is molecular weight standard. Based on the protein profiles, AAC1 is composed of approximately 3 times more protein than a homemade product. Proteins in the homemade product displayed a wider range of molecular weight distribution, containing both large (up to about 300-500 kD) and small proteins (about 5-15kD). By contrast, the majority (i.e., greater than 50%) of proteins in AAC1 has molecular weight of between 70 kDa and 15 kDa.

Individual amino acid content was also analyzed. Table 3 below shows the individual amino acid content of the broth compositions prepared according to the disclosed methods as compared to those prepared using home-style methods as well as other commercial products.

**Table 3 Amino acid composition of different broths**

| **Table Two: Content values above calculated to 100% solids basis:** | 3823 | Commercial product | Home-made-1 | Home-made-2 | AAC1-1 | AAC1-2 | AAC1-3 |
|---|---|---|---|---|---|---|---|
| - | W/W% | % | | | | | |
| TAURINE | 2.50 | | | | | | |
| ASPARTIC ACID | 3.63 | 2.44 | 3.63 | 3.47 | 7.04 | 5.34 | 5.38 |
| THREONINE | 1.44 | 0.76 | 1.52 | 1.42 | 1.75 | 2.13 | 1.79 |
| SERINE | 1.56 | 1.34 | 1.96 | 1.89 | 2.08 | 1.88 | 2.40 |
| GLUTAMIC ACID | 10.25 | 6.98 | 9.44 | 9.26 | 10.52 | 9.50 | 10.14 |
| PROLINE | 3.53 | 3.05 | 5.93 | 5.57 | 9.59 | 10.66 | 11.07 |
| HYDROXYPROLINE | 2.19 | 2.99 | 5.31 | 4.92 | na | 7.00 | 9.39 |
| GLYCINE | 5.97 | 7.38 | 8.79 | 9.47 | 18.02 | 15.41 | 17.68 |
| Lanthionine | 0.06 | 0.00 | 0.00 | 0.00 | 0.00 | 0.00 | 0.00 |
| ALANINE | 3.91 | 3.37 | 4.60 | 4.58 | 8.06 | 7.53 | 7.53 |
| CYSTINE | 0.44 | 0.29 | 0.34 | 0.40 | 0.00 | 0.25 | 0.17 |
| VALINE | **1.44** | **0.73** | **1.43** | **1.42** | **2.44** | **2.19** | **2.21** |
| ISOLEUCINE | **1.13** | **0.47** | **1.18** | **1.02** | **1.54** | **1.59** | **1.59** |
| LEUCINE | **2.72** | **1.51** | **2.48** | **2.32** | **3.38** | **3.47** | **3.43** |
| METHIONINE | 0.81 | 0.52 | 0.93 | 0.77 | 1.01 | 1.06 | 1.10 |
| TYROSINE | 0.88 | 0.44 | 0.81 | 0.65 | 1.09 | 1.00 | 0.90 |
| PHENYLALANINE | 1.00 | 0.73 | 9.53 | 7.03 | 2.30 | 2.16 | 1.98 |
| HYDROXYLYSINE | 0.16 | 0.00 | 0.00 | 0.00 | 0.00 | 0.00 | 0.00 |
| HISTIDINE | 2.03 | 2.56 | 2.70 | 1.73 | 1.32 | 1.16 | 1.00 |
| ORNITHINE | 2.25 | 0.00 | 0.00 | 0.00 | 0.00 | 0.00 | 0.00 |
| LYSINE | 3.75 | 2.67 | 3.07 | 3.00 | 3.96 | 3.66 | 3.05 |
| ARGININE | 3.06 | 2.79 | 4.01 | 3.75 | 8.24 | 6.69 | 6.61 |
| TRYTOPHAN | 0.16 | 0.06 | 0.12 | 0.09 | | 0.22 | 0.15 |
| **Total** | 54.84 | 41.08 | 67.80 | 62.79 | 82.35 | 82.88 | 87.55 |

### Example 4 Comparing levels of chondroitin sulfate (CS) in different broths

Chondroitin sulfate (CS) is an important structural compound found in cartilage and is implicated in joint health. CS has been shown to reduce the levels of many inflammatory mediators such as iNOS, PGE2, COX-2 (Gwendolyn, Spine 2011), and NFkB (Vallières, Osteoarthritis Cartilage, 2010). The goal of this study was to determine the amount of CS in various chicken broth samples. Chicken broths were analyzed for total CS using enzymatic digestion and LC-UV detection (Ji, Journal of AOAC International, 2007). Results were calculated as area on the curve compared to standard samples and the limit of quantification as 8 mg chondroitin sulfate/g dry material (Table 4). Among all chicken broths tested, AAC1 had the greatest amount of CS (8.797% w/w).

**Table 4 Amounts of chondroitin sulfate (CS)**

| Samples | Average (mg) | Dry mg/g | Dry % w/w |
|---|---|---|---|
| Powdered Chicken Broth AAC1 | 17.480 | 87.971 | 8.797 |
| Powdered Chicken Broth H814 | 15.443 | 67.553 | 6.755 |
| Frozen Concentrate TSN | 10.886 | 52.059 | 5.206 |
| Frozen Concentrate IDF Chicken 823 | 8.627 | 42.104 | 4.210 |
| Home Style Broth from Back Bones | 5.866 | 28.258 | 2.826 |
| Frozen Concentrate Turkey 824 | 5.243 | 26.227 | 2.623 |
| Powdered Chicken Broth HML 34511 | 4.979 | 24.335 | 2.434 |
| Powdered Chicken Broth A1004 | 3.817 | 18.305 | 1.830 |
| Powdered Chicken Broth P1301 | 3.075 | 15.054 | 1.505 |
| Home Style Broth from Chicken Parts | 2.185 | 10.99 | 1.09 |
| Home Style Broth from Chicken Necks | 1.937 | 9.361 | 0.936 |

### Example 5 Comparison of concentration of polyphenols in different chicken broths

The concentration of polyphenols in different chicken broths was determined using a modified Folin-Ciocalteau method (Slinkard, American Journal of Enology and Viticulture, 1977). Polyphenols are a class of chemical compounds known to have anti-oxidant and anti-inflammatory properties. While both AAC1 and home-style broths contained polyphenols, AAC1 contained 4828.8 µg/mL GAE of polyphenols, which is approximately 7 times more GAE of polyphenols than a homemade broth (687.7 µg/mL GAE) made from the same kind of chicken parts.

### Example 6 Effects of broth feeding on microbiome in the lower GI

Various broth compositions were fed to rodents and fecal samples were collected at Day 0 (basal) and Day 14. Denaturing gradient gel electrophoresis (DGGE) was used to analyze the changes in the microbial profiles. Broth compositions tested include: the disclosed broth AAC1, water (as negative control), homemade-styled broth and a commercial broth product. The DGGE profiles of the microbiome at Day 0 and Day 14 after the animals were fed different broth compositions were compared.

All of the basal samples showed a relatively high degree of similarity. At most, there was a 9% difference between the basal samples of the AAC1 and water groups. The mean difference between all basal samples was 6.1%. The introduction of the various chicken broths, as well as the two-week time period, resulted in additional variability between the samples. At most, AAC1 and the commercial broth product differed by 15%. In contrast, Homemade broth differed from water and the commercial broth by 9%. The mean difference between all samples was 12%.

All of the samples changed in composition between the basal Day 0 and Day 14 time points. For instance, the difference between the water control on Day 0 (basal) and Day 14 was 7%. This 7% change demonstrated a relatively stable gut microbiome over a period of two weeks and serves as an excellent control. The average variance among all samples from the basal to day 14 time point was 13.5%.

The different profiles were grouped and classified based on banding similarities (i.e., presence/absence of DNA bands) in DGGE. The animals receiving AAC1 chicken broth experienced a distinct shift in their gut microbiomes when compared to animals receiving water or the other two broths. This result is most clearly demonstrated in Figure 1, as hierarchical clustering reveals that the dissimilarities observed in the AAC1 fed animals are unique to this administration. The animals fed AAC1 at day 14 do not cluster with any of the basal or other day 14 groups. This indicated that the AAC1 broth causes a unique shift in the rodent gut microbiome. The scale at the bottom of Figure 1 indicates percent similarity between clusters.

### Example 7 Effects of broth feeding on microbiome in the cecum

Caecal samples for each feeding group were also collected at Day 14 and were analyzed according to the same protocols described above. Although many microorganisms that are found in the feces are also found in the cecum, the contents in the cecum offer an additional look into the gut microbiome and any associated changes that occur within the animal due to the different function/conditions of the organ. The downside to caecal analysis is that the animal must be sacrificed for collection, and therefore no basal samples were taken. DGGE analysis from caecal materials reveals a high degree of similarity between the water, homemade and commercial broth fed animals (average 4.6% difference among all three samples). Interestingly, the AAC1 group was over 14% dissimilar from the water, homemade and the commercial broth group.

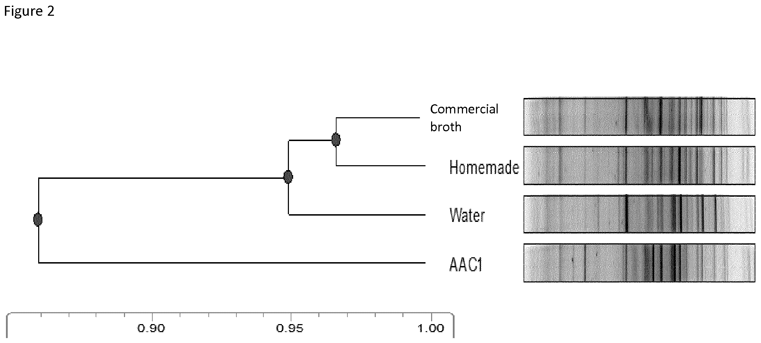

The different profiles were grouped and classified based on banding similarities (i.e., presence/absence of bands) in DGGE. The scale at the bottom of Figure 2 indicates percent similarity between clusters.

Results from analysis of the caecal materials mirror those of the fecal samples. While no baseline information as to the composition of the caecal microbiota is available, 14 days of ingesting the AAC1 broth produced a unique caecal composition (Figure 2).

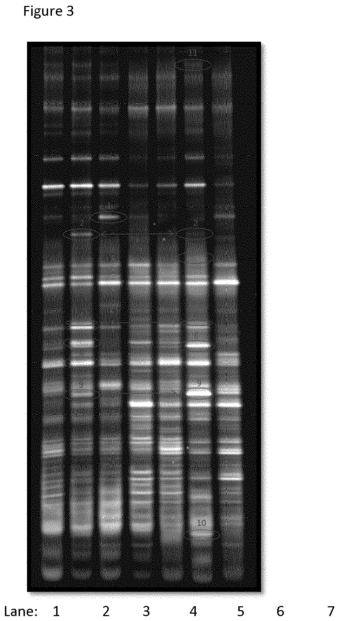

In an effort to compare shifts between fecal and caecal materials, the gel of Figure 3 was created. This gel allowed for the direct comparison across all caecal and select fecal samples. From this result, eleven regions of interest were selected, each corresponding to a unique bacterial species. Direct sequencing of the DNA from the bands allowed for the identification of several species that are altered as a result of AAC1 ingestion (Table 5). Due to the tremendous diversity of the gut microbiota, several of the species could not be identified, as they have never been previously characterized.

**Table 5 Results from DNA sequencing of bands altered by AAC1 feeding**

| **Band** | **Identity** | **Relationship to AAC1 Treatment** | **Summary of Species** | **Phylum** |
|---|---|---|---|---|
| 1 | *Acholeplasma sp.* | Found in all fecal and caecal samples, but appears to be decreased in AAC1 feces. | Saprotrophic or pathogenic. Little known about the role in the gut | Firmicutes |
| 2 | Uncultured Lachnospiraceae | Unique to AAC1 fecal and caecal samples. Not found in any other treatment groups or controls. | Closely related to *Clostridium.* | Firmicutes |
| 3 | *Lactobacillus rhamnosus* | Unique to , AAC1 cecum. Not found in any other samples, fecal or caecal. | Popular probiotic. Certain strains have been shown to produce a number of beneficial health effects. | Firmicutes |
| 4 | Uncultured Lachnospiraceae | Unique to AAC1 fecal and caecal samples. Not found in any other treatment groups or controls. | Closely related to *Clostridium.* | Firmicutes |
| 5 | Mixed Sample/No known sequence | N/D | N/A | N/A |
| 6 | Uncultured *Clostridium sp.* | Unique to AAC1 cecum. Not found in any other samples, fecal or caecal. | Common residents of the gut microbiota. Metabolically diverse. | Firmicutes |
| 7 | Mixed Sample/No known sequence | N/D | N/A | N/A |
| 8 | Uncultured Clostridiales | Found in all fecal and caecal samples, but is apparently increased in AAC1 feces. | Common residents of the gut microbiota. Metabolically diverse. | Firmicutes |
| 9 | Mixed Sample/No known sequence | Unique to AAC1 fecal and caecal samples. Not found in any other treatment groups or controls. | N/A | N/A |
| 10 | Mixed Sample/No known sequence | Unique to AAC1 cecum. Not found in any other samples, fecal or caecal. | N/A | N/A |
| 11 | *Clostridium polysaccharolyticum* | Present in all fecal samples, but also appears in AAC1-treated cecum. Does not appear in any other caecal samples. | Able to degrade a wide variety of complex polysaccharides, including cellulose and xylan. | Firmicutes |

A sequence that is of special interest is band three, which was identified as *Lactobacillus rhamnosus.* This species was only found in the caecal contents of the animals receiving AAC1 broth. *L. rhamnosus* is a popular, commercially-available probiotic, but is also a native resident of the gut microbiota. Several studies have indicated that there are numerous health benefits associated with the ingestion of certain strains of *L. rhamnosus.* For example, research has shown that ingestion of *L. rhamnosus* by mice can lead to lower levels of anxiety and stress hormones.

### Example 8 Use of next generation sequencing to identify probiotic bacteria in fecal samples

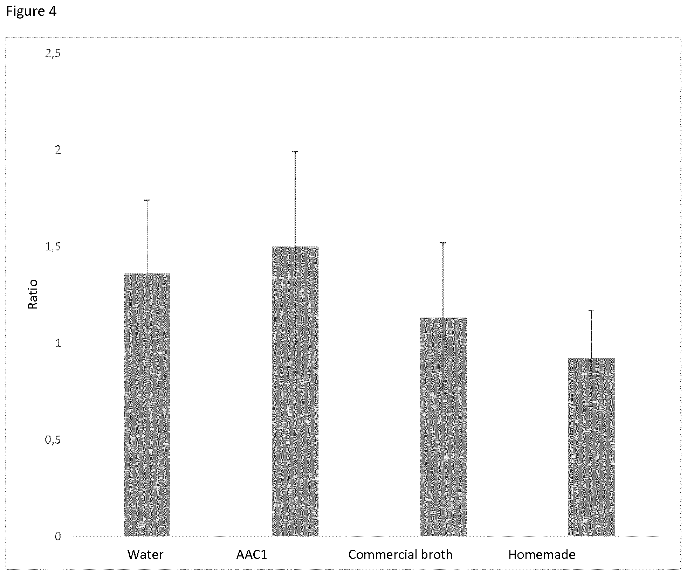

In a healthy human adult, 80% of the identified fecal microbiota may be classified into three dominant phyla: Bacteroidetes, Firmicutes and Actinobacteria. The Firmicutes to Bacteroidetes ratio has been regarded as a good indicator of gut microbiota composition and balance. See Ley et al. (2006). The Firmicutes to Bacteroidetes ratio was measured for the groups of rodents fed with different broth products. As shown in Figure 4, the Firmicutes to Bacteroidetes ratio did not change significantly among the different groups.

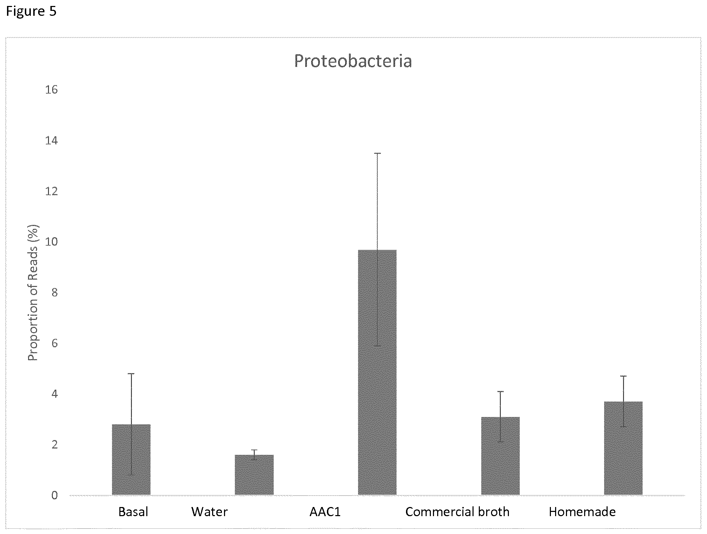

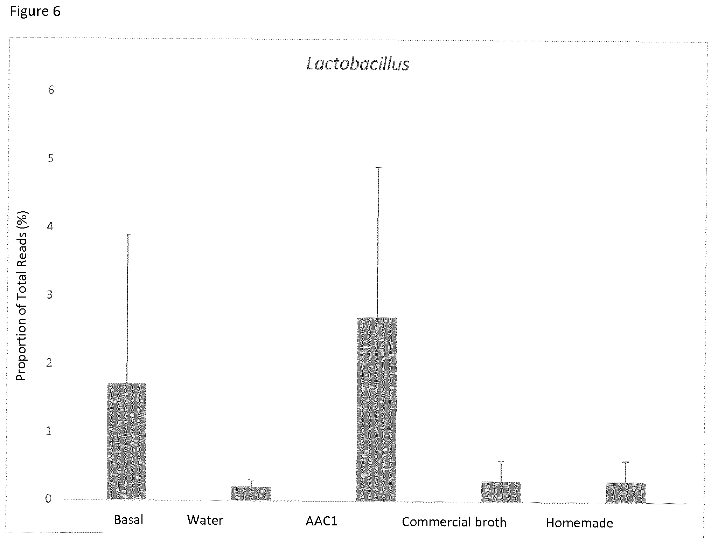

The profiles of fecal microbiota serve as good indicators of microbiota in the lower GI tract. Figure 5 showed the relative amount of Proteobacteria in the lower GI tract of animals fed with different broth compositions. AAC1 selected for Proteobacteria in the lower GI tract of the animals. The relative amounts of *Lactobacillus* in the lower GI tract of the animals fed with different broth compositions were also measured. As shown in Figure 6, AAC1 selected for (or specifically maintained) *Lactobacillus* in the lower GI tract of the animals. In addition, one genus, *Faecalibacterium,* was only found in the group fed with AAC1 for 14 days, but not in other groups or in the basal measurement. Three genera, *Parasutterella, Morganella* and *Oscillospira,* were present at significantly higher levels after 14 days on AAC1 as compared to the basal level.

### Example 9 Use of next generation sequencing to identify probiotic bacteria in caecal samples

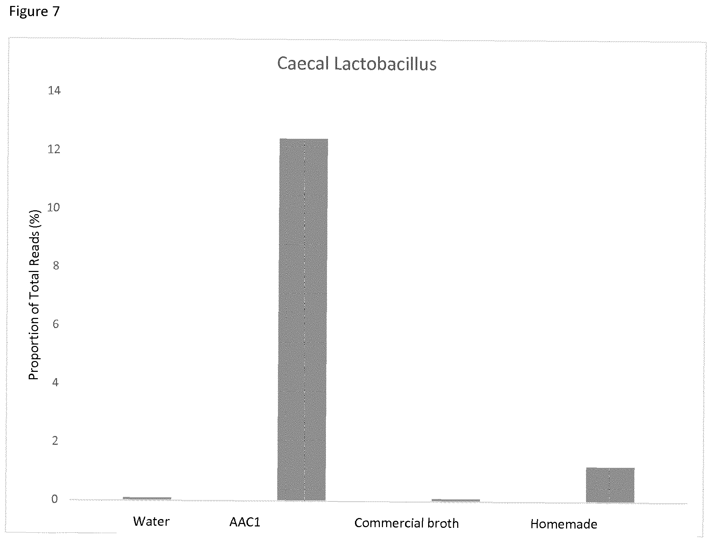

The profiles of caecal microbiota in the groups of rodents fed with different broth products were measured. As shown in Figure 7, AAC1 selected for (or specifically maintained) *Lactobacillus* in the cecum of the animals. In addition, four genera, *Odoribacter, Gastranaerophilales, Faecalibacterium* and *Allabaculum,* were only found in the group fed with AAC1 for 14 days, but not in other groups or in the basal measurement. Three genera, *Lactobacillus, Coprococcus* and *Oscillibacter,* were present at significantly higher levels after 14 days on AAC1 as compared to the basal level. One genus, *Intestimonas,* was present at significantly lower levels after 14 days on AAC1 as compared to the basal level.

### Example 10 The disclosed composition enhance biodiversity in the gut

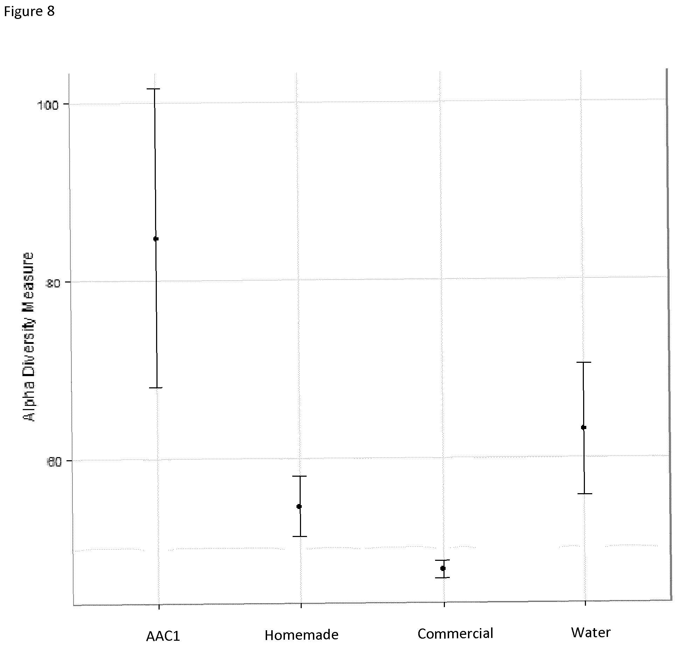

Another important consideration in NGS data analysis is biodiversity. It is generally agreed upon in the literature that a more diverse gut microbiota is beneficial. Two of the most commonly used measurements of biodiversity in NGS analysis are the Chao and Shannon-Weiner indices. Each index produces a value that is representative of how diverse a microbiota is. Figure 8 demonstrates that the degree of diversity after 14 days was much higher in animals fed AAC1 as compared to the diversity in groups fed Homemade, another commercial product and water control groups.

All animal studies described herein were performed using approved protocols in compliance with government rules and regulations.

### References

Arumugam, M., et al. "Enterotypes of the human gut microbiota." Nature (2011).
Bravo, J. A., et al. "Ingstion of Lactobacillus strain regulates emotional behavior and central GABA receptor expression in a mouse via the vagus nerve." Proceedings of the National Academy of Science (2011): doi: 10.1073/pnas. 1102999108.
Larsen, N., et al. "Gut Microbiota in Human Adults with Type 2 Diabetes Differs from Non-Diabetic Adults." PLOS One (2010).
Ley, R. E., et al. "Obesity alters gut microbial ecology." Proceedings of the National Academy of Science (2005): 11070-11075.
Mariat, D., et al. "The Firmicutes/Bacteroides ratio of the human microbiota changes with age." BMC Microbiology (2009).
Ley RE, Tumbaugh P, Klein S, Gordon JI: Microbial ecology: human gut microbes associated with obesity. Nature 2006, 444:1022-1023.

## Claims

1. A composition for use in a method of providing health benefits to an individual by enhancing the microbiome of said individual by enhancing one or more probiotic bacteria in said individual, the method comprising administering to said individual a composition in an amount effective in enhancing said one or more probiotic bacteria, **characterized in that** said composition comprises soluble collagen fiber prepared from poultry, wherein said composition is obtainable by a method comprising the steps of mechanically separating and comminuting poultry parts with bones and cartilage to fine pieces of less than 5 mm, and cooking the pieces at a temperature from 70 to 150 °C for a period from 8 minutes to 6 hours or longer.

2. The composition for the use of claim 1, wherein said small mechanically processed poultry parts are smaller than 4 mm, 3 mm, 2 mm or 1 mm.

3. The composition for the use of any one of the preceding claims, wherein said composition comprises at least 40% protein by weight of total solids.

4. The composition for the use of any one of the preceding claims, wherein said composition comprises less than 10% carbohydrate by weight of total solids.

5. The composition for the use of any one of the preceding claims, wherein said method comprises that the composition is administered to said individual at said effective amount each day for at least 14 days.

6. The composition for the use of any one of the preceding claims, wherein said amount effective in enhancing said one or more probiotic bacteria is the amount of said composition that increases the live count of said one or more probiotic bacteria in the gastrointestinal system of said individual when administered to said individual.

7. The composition for the use of any one of the preceding claims, wherein said composition increases microbial diversity in the gastrointestinal system of said individual.

8. The composition for the use of any one of the preceding claims, the method further comprising identifying an individual in need of increased count of said one or more probiotic bacteria.

9. The composition for the use of any one of the preceding claims, the method further comprising measuring the live count of said one or more probiotic bacteria in the gastrointestinal system of said individual after administering the composition to the individual at said effective amount each day for at least 14 days.

10. The composition for the use of any one of the preceding claims, wherein said one or more probiotic bacteria comprise a lactic acid producing bacterium, especially a *Lactobacillus* bacterium, especially *Lactobacillus rhamnosus.*

11. The composition for the use of any one of the preceding claims, wherein said composition increases the live count of said one or more probiotic bacteria in the gastrointestinal system of said individual as compared to a comparable individual not receiving said composition, said one or more probiotic bacteria comprising at least one member selected from the group consisting of *Parasutterella, Morganella, Oscillospira, Faecalibacterium* and combination thereof.

12. The composition for the use of any one of the preceding claims, wherein said composition increases the live count of said one or more probiotic bacteria in the upper and/or middle sections of the intestine of said individual, said at least one probiotic bacterium comprising at least one member selected from the group consisting of *Lactobacillus, Coprococcus, Oscillibacter, Odoribacter, Gastranaerophilales, Faecalibacterium, Allabaculum,* and combination thereof.

13. The composition for the use of any one of the preceding claims, wherein said method comprises that the composition is administered to the individual as a mixture with one or more probiotic bacteria.

14. The composition for the use of any one of the preceding claims, wherein said composition comprises amino acids glycine, proline and hydroxyproline, wherein glycine constitutes at least 12% (w/w), proline constitutes at least 8% (w/w), and hydroxyproline constitutes at least 7% (w/w) of total amino acids of said composition.

15. The composition for the use of any one of the preceding claims, wherein said composition does not contain detectable amount of gluten.

## Patentansprüche

1. Zusammensetzung zur Verwendung in einem Verfahren zum Bereitstellen von gesundheitlichen Vorteilen für ein Individuum durch Fördern des Mikrobioms des Individuums durch Fördern eines oder mehrerer probiotischer Bakterien in dem Individuum, wobei das Verfahren das Verabreichen einer Zusammensetzung in einer Menge an das Individuum umfasst, die wirksam ist, um das eine oder die mehreren probiotischen Bakterien zu fördern, **dadurch gekennzeichnet, dass** die Zusammensetzung lösliche Kollagenfasern umfasst, die aus Geflügel hergestellt sind, wobei die Zusammensetzung durch ein Verfahren erhältlich ist, das die Schritte des mechanischen Trennens und Zerkleinerns von Geflügelteilen mit Knochen und Knorpel in feine Stücke von weniger als 5 mm und des Kochens der Stücke bei einer Temperatur von 70 bis 150 °C für einen Zeitraum von 8 Minuten bis 6 Stunden oder länger umfasst.

2. Zusammensetzung zur Verwendung gemäß Anspruch 1, worin die kleinen, mechanisch verarbeiteten Geflügelteile kleiner als 4 mm, 3 mm, 2 mm oder 1 mm sind.

3. Zusammensetzung zur Verwendung gemäß einem der vorstehenden Ansprüche, wobei die Zusammensetzung mindestens 40 Gew.-% Protein, bezogen die Gesamtfeststoffe, umfasst.

4. Zusammensetzung zur Verwendung gemäß einem der vorstehenden Ansprüche, wobei die Zusammensetzung weniger als 10 Gew.-% Kohlenhydrate, bezogen auf die Gesamtfeststoffe, umfasst.

5. Zusammensetzung zur Verwendung gemäß einem der vorstehenden Ansprüche, wobei das Verfahren umfasst, dass die Zusammensetzung dem Individuum jeden Tag in der effektiven Menge für mindestens 14 Tage verabreicht wird.

6. Zusammensetzung zur Verwendung gemäß einem der vorstehenden Ansprüche, wobei die Menge, die bei der Fördern des einen oder der mehreren probiotischen Bakterien wirksam ist, die Menge der Zusammensetzung ist, die die Lebendzahl der einen oder mehreren probiotischen Bakterien im Magen-Darm-System des Individuums erhöht, wenn sie an das Individuum verabreicht wird.

7. Zusammensetzung zur Verwendung gemäß einem der vorstehenden Ansprüche, wobei die Zusammensetzung die mikrobielle Vielfalt im Magen-Darm-System des Individuums erhöht.

8. Zusammensetzung zur Verwendung gemäß einem der vorstehenden Ansprüche, wobei das Verfahren ferner das Identifizieren eines Individuums umfasst, das eine erhöhte Anzahl des einen oder der mehreren probiotischen Bakterien benötigt.

9. Zusammensetzung zur Verwendung gemäß einem der vorstehenden Ansprüche, wobei das Verfahren ferner das Messen der Lebendzahl des einen oder der mehreren probiotischen Bakterien im Magen-Darm-System des Individuums umfasst, nachdem die Zusammensetzung dem Individuum jeden Tag in der effektiven Menge für mindestens 14 Tage verabreicht wurde.

10. Zusammensetzung zur Verwendung gemäß einem der vorstehenden Ansprüche, wobei das eine oder die mehreren probiotischen Bakterien ein Milchsäure produzierendes Bakterium, insbesondere ein Lactobacillus-Bakterium, insbesondere *Lactobacillus rhamnosus,* umfassen.

11. Zusammensetzung zur Verwendung gemäß einem der vorstehenden Ansprüche, wobei die Zusammensetzung die Lebendzahl des einen oder der mehreren probiotischen Bakterien im Magen-Darm-System der Person im Vergleich zu einer vergleichbaren Person, die die Zusammensetzung nicht empfängt, erhöht, wobei das eine oder die mehreren probiotischen Bakterien mindestens ein Element umfassen, ausgewählt aus der Gruppe bestehend aus *Parasutterella, Morganella, Oscillospira, Faecalibacterium* und einer Kombination derselben.

12. Zusammensetzung zur Verwendung gemäß einem der vorstehenden Ansprüche, wobei die Zusammensetzung die Lebensdauer des einen oder der mehreren probiotischen Bakterien im oberen und/oder mittleren Abschnitt des Darms des Individuums erhöht, wobei das mindestens eine probiotische Bakterium mindestens ein Element umfasst, das ausgewählt ist aus der Gruppe bestehend aus *Lactobacillus, Coprococcus, Oscillibacter, Odoribacter, Gastranaerophilales, Faecalibacterium, Allabaculum* und einer Kombination derselben.

13. Zusammensetzung zur Verwendung gemäß einem der vorstehenden Ansprüche, wobei das Verfahren umfasst, dass die Zusammensetzung dem Individuum als Mischung mit einem oder mehreren probiotischen Bakterien verabreicht wird.

14. Zusammensetzung zur Verwendung gemäß einem der vorstehenden Ansprüche, wobei die Zusammensetzung die Aminosäuren Glycin, Prolin und Hydroxyprolin umfasst, worin Glycin mindestens 12% (w/w), Prolin mindestens 8% (w/w) und Hydroxyprolin mindestens 7% (w/w) der gesamten Aminosäuren der Zusammensetzung ausmacht.

15. Zusammensetzung zur Verwendung gemäß einem der vorstehenden Ansprüche, worin die Zusammensetzung keine nachweisbare Menge an Gluten enthält.

## Revendications

1. Composition destinée à être utilisée dans un procédé pour fournir des avantages pour la santé d'un individu en améliorant le microbiome dudit individu en améliorant une ou plusieurs bactéries probiotiques chez ledit individu, le procédé comprenant l'administration audit individu d'une composition en une quantité efficace pour améliorer ladite une ou plusieurs bactéries probiotiques, **caractérisé en ce que** ladite composition comprend une fibre de collagène soluble préparée à partir de volaille, dans lequel ladite composition peut être obtenue par un procédé comprenant les étapes consistant à séparer mécaniquement et à broyer des parties de volaille avec des os et du cartilage en morceaux fins de moins de 5 mm, et à cuire les morceaux à une température comprise entre 70 et 150 °C pendant une période comprise entre 8 minutes et 6 heures ou plus.

2. Composition pour l'utilisation de la revendication 1, dans laquelle lesdites petites parties de volaille traitées mécaniquement sont inférieures à 4 mm, 3 mm, 2 mm ou 1 mm.

3. Composition pour l'utilisation de l'une quelconque des revendications précédentes, dans laquelle ladite composition comprend au moins 40 % en poids de protéines par rapport aux solides totaux.

4. Composition pour l'utilisation de l'une quelconque des revendications précédentes, dans laquelle ladite composition comprend moins de 10 % en poids de glucides par rapport aux solides totaux.

5. Composition pour l'utilisation de l'une quelconque des revendications précédentes, dans laquelle ledit procédé comprend le fait que la composition est administrée audit individu à ladite quantité efficace chaque jour pendant au moins 14 jours.

6. Composition pour l'utilisation de l'une quelconque des revendications précédentes, dans laquelle ladite quantité efficace pour améliorer ladite une ou plusieurs bactéries probiotiques est la quantité de ladite composition qui augmente le nombre vivant de ladite une ou plusieurs bactéries probiotiques dans le système gastro-intestinal dudit individu quand elle est administrée audit individu.

7. Composition pour l'utilisation de l'une quelconque des revendications précédentes, dans laquelle ladite composition augmente la diversité microbienne dans le système gastro-intestinal dudit individu.

8. Composition pour l'utilisation de l'une quelconque des revendications précédentes, le procédé comprenant en outre l'identification d'un individu ayant besoin d'un nombre accru de ladite une ou plusieurs bactéries probiotiques.

9. Composition pour l'utilisation de l'une quelconque des revendications précédentes, la méthode comprenant en outre la mesure du nombre vivant de ladite une ou plusieurs bactéries probiotiques dans le système gastro-intestinal dudit individu après administration de la composition à l'individu à ladite quantité efficace chaque jour pendant au moins 14 jours.

10. Composition pour l'utilisation de l'une quelconque des revendications précédentes, dans laquelle ladite ou lesdites bactéries probiotiques comprennent une bactérie produisant de l'acide lactique, en particulier une bactérie *Lactobacillus,* en particulier *Lactobacillus rhamnosus.*

11. Composition pour l'utilisation de l'une quelconque des revendications précédentes, dans laquelle ladite composition augmente le nombre vivant de ladite une ou plusieurs bactéries probiotiques dans le système gastro-intestinal dudit individu par rapport à un individu comparable ne recevant pas ladite composition, ladite une ou plusieurs bactéries probiotiques comprenant au moins un membre choisi dans le groupe consistant en *Parasutterella, Morganella, Oscillospira, Faecalibacterium* et leur combinaison.

12. Composition pour l'utilisation de l'une quelconque des revendications précédentes, dans laquelle ladite composition augmente le nombre vivant de ladite ou desdites bactéries probiotiques dans les parties supérieure et/ou moyenne de l'intestin dudit individu, ladite au moins une bactérie probiotique comprenant au moins un élément choisi dans le groupe constitué par *Lactobacillus, Coprococcus, Oscillibacter, Odoribacter, Gastranaerophilales, Faecalibacterium, Allabaculum et* leurs combinaisons.

13. Composition pour l'utilisation de l'une quelconque des revendications précédentes, dans laquelle ledit procédé comprend le fait que la composition est administrée à l'individu en mélange avec une ou plusieurs bactéries probiotiques.

14. Composition pour l'utilisation de l'une quelconque des revendications précédentes, dans laquelle ladite composition comprend des acides aminés glycine, proline et hydroxyproline, dans laquelle la glycine constitue au moins 12% (p/p), la proline constitue au moins 8% (p/p) et l'hydroxyproline constitue au moins 7% (p/p) des acides aminés totaux de ladite composition.

15. Composition pour l'utilisation de l'une quelconque des revendications précédentes, dans laquelle ladite composition ne contient pas de quantité détectable de gluten.
